# EUROPEAN PATENT APPLICATION

(11) **EP 2 596 759 A1**
(43) Date of publication of application: **29.05.2013**
(21) Application number: 12194099.3
(22) Date of filing: 23.11.2012
(51) Int. Cl.: A61B 18/22

(54) **Laser assisted sclerotherapy method for treating varicose veins**

(30) Priority: 25.11.2011 US 201161563705 P
(71) Applicant: Techlamed S.r.l. Societa' Unipersonale, 50132 Firenze (FI) (IT)
(72) Inventor: Frullini, Alessandro, 50066 Reggello (FI) (IT)
(74) Representative: Bottero, Carlo

(57) **Abstract**

The present invention relates to a laser device including a laser source for generating a pulsed laser radiation having a wavelength of from 1400 to 2300 nm and at least one optical fibre for conveying the generated pulsed laser radiation, for use in a method for treating varicose veins, the laser device being for use in a pretreatment step of a target vein segment thereby causing a substantial diameter reduction of the target vein segment, the pre-treated target vein segment being subsequently subjected to a sclerosing treatment thereby causing occlusion of the vein. The sclerosing agent is preferably a foam sclerosing agent. The above treatment is particularly effective also for larger veins, especially veins exceeding 10 mm of diameter, without causing major discomfort to the patients, and which has a very limited risk of side effects, such as those deriving from the injection of relatively large amounts of sclerosing agent or from damages to the vessel endothelium.

## Description

The present invention relates to a laser assisted sclerotherapy method for treating varicose veins.

Sclerotherapy is a procedure used to treat venous diseases, and particularly chronic venous insufficiency (CVI), wherein the vein segment to be treated is injected with a sclerosant agent by means of a fine needle inserted into the vein in order to chemically ablate the vein segment. The sclerosant agent irritates an internal lining of the vein, causing inflammation, formation of a "sclerus" and subsequently vein transformation in an inner scar that permanently blocks the vein. This technique shows many drawbacks, mainly due to the difficulties encountered in localizing the injected agent at the target treatment site, thereby reducing the effectiveness of the treatment and increasing systemic delivery of the agent (see e.g. US Patent No. 6,726,674).

Foam sclerotherapy (FS) has represented a true revolution for the treatment of CVI. For the first time sclerotherapy was able to achieve very high early occlusion rate of great and lesser saphenous vein (GSV-LSV) with minimal discomfort for the patient. This was particularly true for greater saphenous veins with smaller diameter where the excellent immediate success rate was associated to good medium and late occlusion rate. For larger veins, especially for those exceeding 10 mm of diameter, more controversial data are present in literature but there is agreement that poorer outcome is expected both for immediate result and for late follow up. This is due to the limited volume of sclerosing foam (SF) that can be injected in a single session in order to limit the risk for side effects. In fact there is evidence the some side effects are related to the quantity of extemporary foam used per session as most guidelines limit to 8-10 ml this volume.

Attempts have been made to provide alternative treatments of varicose veins by using a laser beam, in which the laser source has emission characteristics so as to cause a hyalininzing sclerosis in the extracellular matrix of the medium vein coat by photothermal effect, substantially without thermal stress of the morphological components of the tunica media and of the tunica intima (see e.g. US Patent Application No. 2006/0189967). However, such laser technique, although achieving very promising results in restoring vein competency, shows some limitations for therapeutical applications. In other words, the aim of that technique is conservative in order to restore vein competency, and not therapeutic in order to obliterate the diseased vein segment.

The Applicant has now faced the problem of providing a method for treating varicose veins which is effective in obliterating incompetent veins, especially incompetent veins exceeding 10 mm of diameter, without causing major discomfort to the patients, and which has a very limited risk of side effects, such as those deriving from the injection of relatively large amounts of sclerosing agent or from damages to the vessel endothelium.

The Applicant has now found that the above main object and other objects which will be described herein below can be achieved by combining a pre-treatment of the vein segment with a laser radiation having a wavelength of from 1400 to 2300 nm, preferably from 1800 to 2200 nm, which is able to reduce the diameter of the target vein segment, and a subsequent sclerotherapy treatment of the same target vein segment.

According to a first aspect, the present invention therefore relates to a method for treating varicose veins, which comprises:
pre-treating a target vein segment with a pulsed laser radiation having a wavelength of from 1400 to 2300 nm, thereby causing a substantial diameter reduction of the target vein segment;
delivering a sclerosing agent to the pre-treated target vein segment, thereby causing occlusion of the vein.

According to another aspect, the present invention relates to a laser device including a laser source for generating a pulsed laser radiation having a wavelength of from 1400 to 2300 nm and at least one optical fibre for conveying the generated pulsed laser radiation, for use in a method for treating varicose veins, the laser device being for use in a pre-treatment step of a target vein segment thereby causing a substantial diameter reduction of the target vein segment, the pre-treated target vein segment being subsequently subjected to a sclerosing treatment thereby causing occlusion of the vein.

According to a preferred embodiment, the sclerosing agent is a foam sclerosing agent. The foam sclerosing agent is preferably a sclerosing agent, such as polidocanol or sodium tetradecyl sulphate, which is foamed by immission of a gas (e.g. air or a physiological gas such as carbon dioxide). Alternatively, the sclerosing agent is a liquid sclerosing agent. The method according to the present invention can be identified with the acronym L.A.FO.S. i.e. Laser Assisted Foam Sclerotherapy.

The pre-treatment by pulsed laser radiation is preferably carried out by inserting into the target vein segment a catheter which is able to house the optical fiber for conveying the pulsed laser radiation. The insertion can be performed either percutaneusly or intraoperatively. The laser irradiation usually does not require anesthesia as the procedure is absolutely painless. The same catheter can then be used to deliver the sclerosing agent.

The laser radiation has a wavelength of from 1400 to 2300 nm, preferably from 1800 to 2200 nm. Particularly preferred is a holmium laser (HL), having a wavelength of 2100 nm.

Such radiation is preferred since it does not cause coagulation and charring of the blood around the optical fiber tip, thus preventing formation of vapor bubbles in the blood. It is believed that the above advantages are mainly due to the absorption coefficient (α) of water for the above laser wavelengths (more than 2.5 orders of magnitude higher than either the standard laser diodes, or the Nd:YAG lasers). Preferably, the laser radiation has an absorption coefficient (α) of water ranging from 2 cm⁻¹ to 150 cm⁻¹, more preferably from 10 cm⁻¹ to 50 cm⁻¹, measured at 22°C on freshly distilled filtered water, as described, for instance, by Kou, L. et al, Applied Optics, 1 July 1993, Vol. 32. No. 19, 3531-3539. This means that, in a tissue with a high water content, such as blood and vein walls, the energy penetration into the tissue is only limited to the targeted tissue, with no risks of inducing damages to the tissue surroundings, particularly no risks of fibrosis and damages to the saphenous nerve.

In a particularly preferred embodiment, the pulsed laser radiation is a short duration pulse laser radiation, so as to avoid as much as possible heat build-up in the treated vein which may cause damages to the vessel endothelium. Preferably, the short duration pulse radiation has a pulse duration of from 100 µsec to 1000 µsec, more preferably from 200 µsec to 700 µsec. Particularly preferred is a pulse duration of 350 µsec.

Preferably, the laser radiation is pulsed at a frequency of from 1 to 50 Hz, more preferably from 2 to 25 Hz, even more preferably from 5 to 15 Hz.

Preferably, the laser radiation is emitted at an average maximum power of from 0.5 to 20 W, more preferably from 1 to 12 W.

Preferably, each emitted pulse has an energy of from 50 to 2000 mJ, more preferably from 150 to 700 mJ.

It should be noted that the use of a continuous (i.e. not pulsed) laser radiation, with an energy of at least 500 mJ, would cause a heat build-up around the optical fibre tip which can be easily transferred to the surrounding tissues, such as nerves and arteries. Moreover, formation of platelet clots around the optical fibre may cause a local increase of the temperature. Conversely, the use of a pulsed laser radiation as herein described prevents formation of a clot around the optical fibre tip, thus preserving the ability of the blood to flow during the treatment, which allows heat dissipation and thus cooling of the fibre tip. Moreover, the pulsed laser radiation as herein described causes shrinkage of the treated vein segment by acting on the collagen of the median vein coat only, without inducing any modifications in the (inner) endothelium and/or in the heat wall adventitia, thereby limiting the risk of nerve damages.

The pre-treatment step with pulsed laser radiation produces an immediate and significant reduction of vein diameter transforming the large vessel in a smaller one, that can be treated with better chance of success with a limited dose of sclerosing agent, particularly of foam sclerosing agent. More specifically, the Applicant has surprisingly found that the pre-treatment step with laser radiation as described above decreases the vein size by up to 3 times and this causes the decrease of the volume of blood flowing in the vein by up to 9 times. The dramatic reduction of the volume of the blood flowing in the vein and the conservation of the endothelium integrity make easier, faster and safer the sealing of the vein, induced by the sclerosant injection. Moreover, the coarctation of the vein walls, which is advantageously induced especially in the short pulse duration mode, makes the vein size be more even. Consequently, the optical fibre is substantially coaxial within the target vein segment and, therefore, more equidistant from the vein walls. This is an advantage because the laser beam is safer and faster, without requiring the use of more complexes, invasive and expensive catheters. Such catheters are, in fact, big in size and then greatly invasive and cumbersome to be inserted into the vein, and they would make the treatment of the venous trunk below the knee impossible, or at least extremely difficult.

Both greater and lesser saphenous veins (GSV-LSV) are suitable for the method according to the present invention, which anyway can also be used for veins different from the saphenous veins and for vascular malformations.

The following clinical studies were carried out.

For a correct diagnosis, the patients were studied before the procedure with clinical and sonographic evaluation in standing position. Reflux was considered as positive when exceeded 1 second and was elicited using a manual compression/release manoeuvre on the calf.

With the patient in lying position with the thorax slightly elevated (10-15°) we gained access to the vein using a simple vascular cannula 17G or with another equivalent device suitable to gain vascular access positioned with ultrasound guidance. With the cannula firmly secured to the skin a 5 ml syringe with saline solution was attached and the cannula flushed. The HL fiber was then entered into the cannula and fiber position was checked with the duplex. The tip of the cannula was placed 1-2 cm below the sapheno-femoral or the sapheno-popliteal junction and manually retracted while delivering energy. Echographic monitoring of the vein was used to check diameter reduction and duration of laser procedure was recorded. Fluency levels were in the range of 100-500 mJ. The laser system consisted in a Ho:YAG laser (wavelength: 2100 nm) with 5 W of maximum power and pulse frequency of 10 Hz. It is believed that HL is able to shrink collagen fibres of the tunica media, reducing vein diameter size, but without damaging the endothelium of the vessel itself.

It is worthwhile to point out that, with current Endovenous Laser Ablation (EVLA) technique, which is used to treat varicose veins, the laser beam usually spreads light even out of the wall vein, potentially affecting also saphenous nerve and other perivenous structures. For that reason the EVLA procedure is always carried out under tumescent solution injection. This solution is a mixture of physiological sterile solution and local anesthetic drug. This solution is injected, before EVLA, surrounding the saphenous vein. The problem is that, after this injection, the patient loses his/her physiological feedback. Conversely, with the method according to the present invention, the laser beam does not reach the out of the vein, therefore it is not necessary to use tumescent solution and the patient keeps its sensitivity, thus, in the case the treatment becomes intolerable or a perivenous structure is going to be damaged, the operator can immediately stop it.

At the end of the laser pre-treatment, we used the same cannula to inject a sclerosant foam (SF) produced, e.g., with polidocanol or sodium tetradecyl sulphate using the three way technique with 10 complete passages or alternative technique in order to produce a sclerosing foam. After foam injection the patient was asked to lie down for 3 minutes and a medical degree stocking is positioned on the limb. An eccentric compression using a pad was used at the thigh level for the first 48 hours. The garment was prescribed continuously for 48 hours then only day time for one month. We asked the patient to then walk for 30 minutes after discharge and to walk daily for at least one hour in the treatment period. The patients were the checked clinically and with duplex on regular basis for the first month and recheck were scheduled at 3 and 6 months.

Echographic appearance of the vein wall demonstrated wide thickening in association to external and internal diameter reduction. This effect was easily monitored with ultrasound study and the desired final diameter could be achieved simply maintaining a longer energy delivering.

No significant pain is usually reported during procedure apart from occasional discomfort sensation which never needed conversion to local anesthesia. The pre-treatment step by pulsed laser radiation was useful in order to obtain better immediate results, less chance of complications (due to the minor volume of SF injected), and possibly better medium and long time results. In comparison with thermal ablation obtained with laser, radiofrequency, steam or alternative methods, this procedure has different aims (the purpose is shrinkage, not ablation) and carries much lower chance of complications. This is due to the extremely reduced energy delivered which permits accumulation of heat mostly in the tunica media. This means almost zero chance of thermal damage of the structures surrounding the target vein, a typical complication of laser or radiofrequency thermal ablation. Another advantage of the method according to the present invention is a reduced "downtime" for the patients, namely the time that elapses between the treatment and the patient's complete healing is remarkably reduced.

## Claims

1. A laser device including a laser source for generating a pulsed laser radiation having a wavelength of from 1400 to 2300 nm and at least one optical fibre for conveying the generated pulsed laser radiation, for use in a method for treating varicose veins, the laser device being for use in a pre-treatment step of a target vein segment thereby causing a substantial diameter reduction of the target vein segment, the pre-treated target vein segment being subsequently subjected to a sclerosing treatment thereby causing occlusion of the vein.

2. The laser device according to claim 1, wherein the sclerosing agent is a foam sclerosing agent.

3. The laser device according to claim 1, wherein the sclerosing agent is a liquid sclerosing agent.

4. The laser device according to claim 1, wherein the pre-treatment by pulsed laser radiation is carried out by inserting into the target vein segment a catheter which is able to house the optical fiber for conveying the laser radiation.

5. The laser device according to claim 4, wherein said catheter is used also to deliver the sclerosing agent.

6. The laser device according to claim 1, wherein the pulsed laser radiation has a wavelength of from 1800 to 2200 nm, preferably is a holmium laser (HL) radiation having a wavelength of 2100 nm.

7. The laser device according to claim 1, wherein the pulsed laser radiation has an absorption coefficient (α) of water ranging from 2 cm⁻¹ to 150 cm⁻¹, preferably from 10 cm⁻¹ to 50 cm⁻¹.

8. The laser device according to claim 1, wherein the pulsed laser radiation is a short duration pulse laser radiation.

9. The laser device according to claim 1, wherein the pulsed laser radiation has a pulse duration of from 100 µsec to 1000 µsec, preferably from 200 µsec to 700 µsec, more preferably of 350 µsec.

10. The laser device according to claim 1, wherein the laser radiation is pulsed at a frequency of from 1 to 50 Hz, more preferably from 2 to 25 Hz, even more preferably from 5 to 15 Hz.

11. The laser device according to claim 1, wherein the pulsed laser radiation is emitted at an average maximum power of from 0.5 to 20 W, preferably from 1 to 12 W.

12. The laser device according to claim 1, wherein each emitted pulse has an energy of from 50 to 2000 mJ, more preferably from 150 to 700 mJ.
